# DEMANDE DE BREVET EUROPEEN

(11) **EP 1 120 466 A2**
(43) Date de publication de la demande: **01.08.2001**
(21) Numéro de dépôt: 01106372.4
(22) Date de dépôt: 14.01.1999
(51) Int. Cl.: C12N 15/86, C12N 5/10, C12N 5/06, A61K 48/00

(54) **Utilisation d'éléments de régulation négative pour l'expression neurospécifique de transgènes**

(30) Priorité: 12.02.1998 FR 9801715
(62) Demande divisionnaire de: 99900923.6
(71) Demandeur: Aventis Pharma S.A., 92160 Antony (FR)
(72) Inventeur: Kiefer, Hélène, 57230 Bitches (FR); Mallet, Jacques, 75013 Paris (FR); Millecamps, Stéphanie, 59223 Roncq (FR)
(74) Mandataire: Bouvet, Philippe

(57) **Abrégé**

La présente invention concerne de nouvelles méthodes et constructions permettant de contrôler l'expression d'un acide nucléique. En particulier, l'invention concerne des méthodes et constructions utilisant des séquences NRSE, permettant d'obtenir une expression ciblée de transgènes dans les cellules nerveuses in vivo ou ex vivo. L'invention est particulièrement adaptée à des applications de transfert de gènes in vivo, pour des approches thérapeutiques ou scientifiques par exemple.

## Description

La présente invention concerne de nouvelles méthodes, des constructions ainsi que des vecteurs contenant ces constructions, permettant de contrôler l'expression d'un acide nucléique. En particulier, l'invention concerne des méthodes, des constructions et des vecteurs permettant d'obtenir une expression ciblée de transgènes dans les cellules nerveuses in vivo ou ex vivo. L'invention est particulièrement adaptée à des applications de transfert de gènes in vivo, pour des approches thérapeutiques ou scientifiques par exemple.

La thérapie génique in vivo ou ex vivo permet un transfert local et efficace de gènes codant pour des facteurs d'intérêt (transgènes), en particulier dans le système nerveux d'un organisme hôte. A cet égard, différents types de vecteurs ont été utilisés avec succès pour le transfert de différents types de transgènes dans les cellules nerveuses in vivo ou ex vivo. On peut citer notamment les vecteurs viraux de type adénovirus, AAV ou HSV, ou certains vecteurs non-viraux de type polymères cationiques (polyéthylène imine par exemple). Ainsi, ces vecteurs ont permis un transfert efficace et stable de transgènes dans des cellules du système nerveux in vivo (WO 94/08026, WO 93/09239, WO 96/02655). La possibilité d'effectuer ce type de transfert offre de nombreuses applications, notamment dans le domaine médical. Ainsi, ces vecteurs sont utilisables dans des approches de thérapie génique in vivo ou ex vivo. A cet égard, différentes études précliniques sont actuellement en cours concernant le transfert de facteurs trophiques dans le système nerveux. Ces vecteurs sont également utilisables pour la création d'animaux transgèniques permettant de tester des composés ou encore pour différentes études de marquage ou de biodisponibilité.

Pour toutes ces applications, même si une bonne efficacité et une bonne stabilité du transfert semblent aujourd'hui atteintes, le contrôle de l'expression des transgènes est un impératif. A cet égard, différents systèmes ont été décrits pour tenter de restreindre l'expression de transgènes à certains tissus seulement, par exemple en utilisant des promoteurs dits "spécifiques de tissus" ou des systèmes chimériques complexes nécessitant l'emploi de plusieurs constructions et/ou de facteurs de régulation. A titre d'exemples de promoteurs spécifiques de tissus, on peut citer notamment le promoteur Glial Fibrillary Acidic Protein, qui est principalement actif dans les cellules nerveuses gliales. Toutefois, les systèmes de régulation disponibles ou validés à ce jour présentent certains inconvénients, et notamment une force et/ou une sélectivité pas toujours satisfaisantes. Ainsi, dans la grande majorité des systèmes connus, la sélectivité s'obtient généralement au détriment de la force du promoteur, et il est donc difficile d'avoir une expression à la fois importante, stable et spécifique.

La présente invention fournit des méthodes, des constructions ainsi que des vecteurs contenant ces constructions, permettant de remédier aux inconvénients de l'art antérieur. En particulier, la présente invention décrit un promoteur chimérique permettant le ciblage neuronal de l'expression. L'invention utilise à cet égard des éléments de régulation négative, empêchant l'expression des gènes dans les cellules non neuronales. Ce type de constructions présente plusieurs avantages par rapport aux systèmes antérieurs. D'une part les séquences de répression et les promoteurs ubiquitaires étant souvent courts et bien caractérisés, on peut facilement les associer à d'autres éléments de régulation. D'autre part ces éléments de régulation peuvent être associés en théorie à n'importe quel promoteur ubiquitaire et représentent donc un système astucieux permettant d'obtenir facilement une expression neurospécifique de transgènes d'intérêt même lorsque le vecteur d'expression utilisé a peu ou pas de tropisme pour les cellules nerveuses.

De nombreux travaux décrivent des séquences cis réprimant la transcription des gènes neuronaux dans des cellules non neuronales. Un type particulier de séquences doué de cette propriété est constitué des NRSE ("Neuron Restrictive Silencer Element"), encore appelé RE-1 (Repressor élément-1). NRSE est une courte séquence, de 21 pb environ, mise en évidence en amont de plusieurs gènes neuronaux : SCG10 [1], canal sodique II [2] et synapsine I [3] par exemple. Par ailleurs, des séquences analogues ont été retrouvées en amont de nombreux autres gènes neuronaux, même si leur fonctionnalité n'a pas toujours été démontrée à ce jour [4]. Ces séquences NRSE répriment l'expression non-neuronale des gènes en liant un facteur de transcription spécifique, la protéine NRSF (Neuron Restrictive Silencer Factor), encore appelée REST (RE-1 Silencing Transcription factor), présente uniquement dans les cellules non neuronales [5]. Cette protéine appartient à la famille des facteurs de transcription à doigts de zinc. Elle intervient au cours du développement pour initier la répression des gènes neuronaux dans les cellules non neuronales puis à l'âge adulte dans le maintien de cette répression.

Les équipes ayant caractérisé la présence des séquences NRSE en amont des gènes neuronaux ont vérifié la capacité de ces séquences à diminuer l'expression de gènes rapporteurs après transfection de cellules non neuronales. Ils ont dans ce but associé leur séquence NRSE à un promoteur hétérologue minimal, c'est-à-dire un promoteur ne permettant qu'une transcription basale. Ainsi, placée en amont du promoteur de la thymidine kinase par exemple, une copie de la séquence NRSE du canal calcique II réduit l'expression du gène rapporteur de moitié dans les cellules non nerveuses [9]. De même, deux copies de la séquence NRSE de la synapsine I associées au promoteur minimal c-fos permettent de réduire de 75% l'expression dans les cellules non-nerveuses [3]. Par ailleurs, la présence d'une séquence NRSE a aussi permis de réguler l'activité du promoteur de SV40 dans les cellules non neuronales [10].

Toutefois, les séquences NRSE n'ont jamais été utilisées pour diriger l'expression d'un promoteur ubiquitaire fort, ni dans une perspective de thérapie génique. Ainsi, il n'a jamais été montré si de telles séquences étaient capables de supprimer l'expression induite par un promoteur fort. De même, il n'a jamais été montré si ces séquences, placées dans un environnement chimérique, étaient toujours actives in vivo. Plus généralement, l'activité de ces séquences combinées à des promoteurs hétérologues n'a jamais été démontrée in vivo jusqu'à présent.

L'invention concerne des acides nucléiques recombinants permettant l'expression neurospécifique de gènes. Elle concerne également les vecteurs comprenant ces acides nucléiques, notamment d'origine virale, ainsi que les cellules contenant ces acides nucléiques et/ou vecteurs. L'invention concerne également des promoteurs chimériques particuliers, adaptés à l'expression neurospécifique de gènes dans le système nerveux in vivo. L'invention concerne encore des compositions comprenant ces éléments, et leur utilisation pour le transfert de gènes.

Un premier aspect de l'invention réside donc dans un acide nucléique recombinant comprenant:
- un promoteur
- une ou plusieurs séquences NRSE, et
- un gène thérapeutique.

Les constructions selon l'invention comprennent donc une région de répression dans les cellules non-neuronales de l'expression active du gène thérapeutique, induite par le promoteur. Cette région de répression est constituée d'un ou plusieurs motifs NRSE.

Le motif NRSE utilisé dans le cadre de la présente invention comprend avantageusement tout ou partie de la séquence de 21 pb suivante : TTCAGCACCACGGAGAGTGCC (SEQ ID n° 1) Cette séquence correspond à la séquence NRSE du gène SCG10 [2]. Néanmoins, il est entendu que le motif NRSE utilisé dans le cadre de l'invention peut comporter certaines variations par rapport à cette séquence, dans la mesure où il conserve les propriétés de répression sus-indiquées. Ainsi, des séquences de type NRSE présentant certaines variations ont été observées dans différents gènes, tels que décrits dans l'article de Schoenherr et al. [4], incorporé à la présente demande par référence. Sur la base des variants observés, une séquence NRSE consensus a été définie, correspondant à la séquence rencontrée le plus fréquemment. Cette séquence est : TTCAGCACCACGGACAGCGCC (SEQ ID n° 2). Des variants préférés au sens de l'invention comprennent des substitutions sur 1 à 5 paires de bases de la séquence SEQ ID n° 2 ci-dessus. Plus préférentiellement, ils comprennent des variations sur 1 à 3 paires de bases. A cet égard, les variations sont préférentiellement localisées sur les résidus 1, 2, 10, 11, 15, 18, 19 et/ou 20 de la séquence ci-dessus. Les substitutions peuvent correspondre à la suppression ou au remplacement de la base concernée par toute autre base. Ainsi, un motif NRSE peut être représenté plus généralement par tout ou partie de la séquence suivante : NNCAGCACCNNGGANAGNNNC (SEQ ID n° 3) dans laquelle N désigne une base choisie parmi A, T, C ou G. Des exemples particuliers de motifs NRSE utilisables dans le cadre de la présente invention sont notamment les suivants :

TTCAGCACCACGGACAGCGCC (SEQ ID n° 2)

TTCAGCACCACGGAGAGTGCC (SEQ ID n° 4)

TTCAGCACCGCGGACAGTGCC (SEQ ID n° 5)

TTCAGCACCTCGGACAGCATC (SEQ ID n° 6)

TTCAGCACCGCGGAGAGCGTC (SEQ ID n° 7)

TCCAGCACCGTGGACAGAGCC (SEQ ID n° 8)

TTCAGCACCGAGGACGGCGGA (SEQ ID n° 9)

ATCAGCACCACGGACAGCGGC (SEQ ID n° 10)

TTCAGCACCTAGGACAGAGGC (SEQ ID n° 11)

En outre, ces motifs peuvent également comporter, à l'une ou l'autre ou aux deux extrémités, des bases supplémentaires n'interférant pas avec l'activité de répression sus-indiquée. En particulier, il peut s'agir de sites de restrictions, de séquences neutres ou de séquences provenant d'étapes de clonage et contenant par exemple des régions d'un plasmide ou d'un vecteur, ou de séquences bordant le motif NRSE dans le gène d'origine.

Ces différents motifs peuvent être préparés par toutes les techniques connues de l'homme du métier pour la préparation d'acides nucléiques. Ainsi, ils peuvent être préparés par les techniques de synthèse automatique d'acides nucléiques en utilisant des synthétiseurs commerciaux. Ils peuvent également être obtenus par criblage de banques d'ADN, par exemple par hybridation avec des sondes spécifiques ou par des expériences de liaison à un facteur NRSF. En outre, tout autre variant de la séquence SEQ ID n° 1 peut être identifié à partir de banques d'ADN par recherche d'homologies par exemple.

Une fois synthétisé, le motif de type NRSE peut ensuite être testé fonctionnellement. Pour cela, un premier test consiste notamment à déterminer la capacité du motif à lier le facteur NRSF. Ceci peut être réalisé dans différentes conditions et par exemple par des expériences de retard de migration sur gel selon la technique décrite par Mori et al. [2] ou par Schoenherr et al. [5], incorporés à la présente par référence. Dans un second temps, la capacité du motif à réprimer l'expression peut être déterminée par insertion de ce motif dans un plasmide test contenant un gène rapporteur (Chloramphenicol Acetyl Transferase, Luciférase, LacZ, etc.) sous le contrôle d'un promoteur, et comparaison des niveaux d'expression dudit gène rapporteur dans des cellules nerveuses et dans des cellules non nerveuses. Ce type de méthodologie est décrit par exemple dans Schoenherr et al. [4,5] ainsi que dans les exemples. Préférentiellement, le motif est considéré comme fonctionnel lorsqu'il génère un différentiel d'expression entre des cellules nerveuses et non-nerveuses de 40% au moins. Plus préférentiellement, ce différentiel est de 50% au moins, avantageusement, de 60% au moins.

Comme indiqué ci-avant, les acides nucléiques de l'invention peuvent comprendre un ou plusieurs motifs NRSE tels que définis ci-dessus. Il peut s'agir soit du même motif répété plusieurs fois, soit de plusieurs variants. Préférentiellement, les constructions de l'invention comprennent le même motif répété plusieurs fois. Les constructions peuvent comprendre jusqu'à 50 motifs. Avantageusement, le motif est répété de 2 à 20 fois, et de préférence de 3 à 15. Comme illustré dans les exemples, des résultats intéressants ont été obtenus avec des répétitions de 3, 6 ou 12 motifs, les résultats étant particulièrement importants avec 6 et 12 répétitions.

Les motifs NRSE peuvent être insérés dans les constructions de l'invention dans toute région non-transcrite ou non traduite ou dans un intron. Avantageusement, ils sont placés dans les régions 5' non-codantes, encore plus préférentiellement dans la région proximale du promoteur. Par ailleurs, l'activité de ces motifs étant indépendante de leur orientation, ils peuvent être insérés dans le sens de la transcription aussi bien que dans l'orientation opposée. Enfin, lorsque plusieurs motifs sont utilisés, ils sont préférentiellement insérés côte-à-côte, en une même région de la construction. Il est entendu qu'ils peuvent toutefois être insérés en des régions différentes.

Le deuxième élément entrant dans la composition des acides nucléiques de l'invention est un promoteur, permettant l'expression du transgène dans la cellule visée. Avantageusement, il s'agit d'un promoteur actif dans les cellules ou tissus nerveux, notamment d'un promoteur eucaryote. A cet égard, il peut s'agir par exemple d'un promoteur ubiquitaire, c'est-à-dire fonctionnel dans la majorité des types cellulaires. Encore plus préférentiellement, il s'agit donc d'un promoteur ubiquitaire eucaryote. Le promoteur peut être autologue, c'est-à-dire provenant de la même espèce que la cellule dans laquelle l'expression est recherchée ou xénogénique (provenant d'une autre espèce). On peut citer à titre d'exemples avantageux des promoteurs ubiquitaires eucaryotes des promoteurs forts tels que le promoteur du gène de la phosphoglycerate kinase 1 (PGK). On entend par promoteur dit fort, tout promoteur dont l'activité est comparable à celle des promoteurs viraux . Chez les eucaryotes, PGK est une enzyme intervenant lors de la glycolyse. Chez la souris, le promoteur de ce gène, d'environ 500 pb, comporte une région dite "enhancer" (-440/-120) et une région promotrice (-120/+80) contenant plusieurs sites d'initiation de la transcription [6]. L'efficacité de ce promoteur PGK a déjà été démontrée dans de précédentes expériences de transfert de gènes in vitro et in vivo [7,8].

Selon un mode de réalisation préféré, l'invention concerne un acide nucléique comprenant le promoteur PGK et une ou plusieurs séquences NRSE. Comme illustré dans les exemples, ce type de construction permet de diriger l'expression neurospécifique d'un transgène. A cet égard, l'invention concerne également un promoteur chimérique comprenant un promoteur ubiquitaire fort et une ou plusieurs séquences NRSE. L'invention montre en effet que ces séquences NRSE peuvent être utilisées pour réprimer efficacement l'activité d'un promoteur fort, y compris in vivo. Ceci rend donc possible l'utilisation de ces promoteurs nouveaux dans des applications nombreuses. Un promoteur chimérique particulier au sens de l'invention est représenté par la désignation xNRSE-PGK dans lequel x est un nombre entier de 1 à 50 et de préférence de 1 à 20.

D'autres promoteurs eucaryotes ubiquitaires utilisables dans le cadre de la présente invention sont par exemple les promoteurs dirigeant l'expression des gènes du métabolisme cellulaire obligatoire (ces gènes sont dits "domestiques" ou "de ménage" et spécifient des protéines nécessaires à des fonctions communes à toutes les cellules). Il s'agit par exemple de gènes intervenant dans le cycle de krebs, dans la respiration cellulaire ou encore dans la réplication ou la transcription d'autres gènes. On peut citer comme exemples particuliers de ce type de promoteurs, le promoteur des gènes α1-antitrypsine, β-actine, vimentine, aldolase A ou Ef1α (facteur d'élongation).

Le promoteur utilisé dans le cadre de l'invention peut encore être un promoteur eucaryote de type neurospécifique, permettant ainsi d'améliorer sa neurospécificité. On peut citer à titre d'exemple le promoteur des gènes NSE (Neuron Specific Enolase), NF (Neurofilament), TH (Tyrosine Hydroxylase), DAT (Dopamine Transporter), chAT (Choline Acetyl Transferase), DBH (Dopamine β-Hydroxylase), TPH (Tryptophane Hydroxylase), GAD (Glutamic Acid Deshydrogenase) et, plus généralement, tous les promoteurs des enzymes de synthèse ou des transporteurs des neuromédiateurs ou tout autre promoteur de gènes dont l'expression est spécifique d'un type ou sous-type neuronal ou glial donné.

Enfin, on peut aussi envisager l'utilisation de promoteurs viraux, tels que par exemple les promoteurs CMV (Cytomégalovirus), RSV (Rous Sarcoma Virus), TK (Thymidine Kinase), SV40 (Simian Virus) et LTR (Long Terminal Repeat).

Par ailleurs, les acides nucléiques de l'invention comprennent également un gène thérapeutique. On entend par gène thérapeutique au sens de la présente invention, tout acide nucléique comprenant au moins une phase ouverte de lecture codant un ARN ou un polypeptide thérapeutique ou vaccinal. L'acide nucléique peut être un ADN complémentaire, génomique, synthétique ou semi-synthétique. Il peut être d'origine variées telles que mammifère, plante, virale, artificielle, etc. Le produit de transcription ou de traduction présente donc des propriétés thérapeutiques ou vaccinales. On peut citer à titre d'exemple particuliers des enzymes, facteurs de croissance (facteurs trophiques notamment), neurotransmetteurs ou leurs précurseurs, des facteurs toxiques (Thymidine Kinase par exemple), des anticorps ou fragments d'anticorps, etc.

L'utilisation des constructions de l'invention peut être envisagée pour diriger l'expression de un ou plusieurs gènes codant un ARN ou une protéine que l'on souhaiterait adresser au neurone sans expression de ceux-ci dans les cellules non-neuronales, dans le but d'établir des modèles animaux ou dans une perspective de thérapie étiologique ou symptomatique, substitutive ou suppressive. Il peut s'agir par exemple de gènes de la famille des facteurs trophiques, tels que par exemple les neurotrophines (NGF, BDNF, NT3, NT4-5, GMF....), les facteurs de croissance [famille des fibroblastes growth factors FGF (a et b), famille des vascular endothelial cell growth factors VEGF, famille des epidermal growth factors EGF, famille des Insulin growth factors IGF (I et II)], la superfamille des TGFβ dont les familles des TGFβ, GDNF/neurturine.

Il peut également s'agir de gènes codant des cytokines, tels que par exemple le CNTF, LIF, Oncostatine M, Cardiotrophine 1, ou des protéines de la famille des interleukines.

Il peut encore s'agir de gènes codant pour les récepteurs de ces différents facteurs ou codant pour les facteurs de transcription régulant l'expression de ces différents facteurs. Il peut également s'agir de gènes codant pour les enzymes de synthèse ou de dégradation des différents neurotransmetteurs et neuropeptides ou de leurs précurseurs, ou des cofacteurs essentiels à cette synthèse ou ce catabolisme, ou encore des facteurs de transcription régulant l'expression de ces protéines; ainsi que de gènes codant pour les récepteurs des neurotransmetteurs/neuropeptides (ou pour des sous-unités de ces récepteurs) et pour les protéines intervenant dans les voies de transduction.

D'autres gènes d'intérêt dans le cadre de l'invention sont notamment des gènes codant pour des agents antioxydants tels que par exemple la SOD (Superoxyde Dismutase), la GPX (Glutathion Peroxydase), la Catalase ou une enzyme de la respiration cellulaire; les enzymes impliquées dans le cycle cellulaire comme p21, ou autres protéines inhibitrices des kinases dépendantes ainsi que les gènes de l'apoptose tels que ICE, Bcl2, BAX,....

De façon plus générale, tout gène dont l'anomalie d'expression induit une pathologie du système nerveux peut être exprimé dans les constructions de l'invention, tels que des gènes dont la mutation est directement à l'origine de pathologies ou des gènes dont les produits interviennent dans la même voie métabolique. Il peut encore s'agir de gènes toxiques, pour la thérapie anticancéreuse (par exemple la Thymidine Kinase ou le Cytosine Déaminase), des ARN antisens, ribozymes, voire des gènes rapporteurs pour les études de développement, cinétique et/ou biodisponibilité, tels que les gènes β-Galactosidase, Luciferase, ou GFP (Green Fluorescent Protein). Il peut également s'agir des gènes impliqués dans les systèmes de recombinaison conditionnelle dans le système nerveux dans le but d'établir des modèles animaux, comme par exemple des animaux transgéniques y compris le système de knock out conditionnel.

Il est entendu que l'homme du métier peut aisément adapter le type de gène en fonction de l'utilisation recherchée.

Dans les acides nucléiques de l'invention, les différents éléments sont agencés de sorte que le promoteur contrôle l'expression du gène thérapeutique et la ou les séquences NRSE contrôlent l'activité du promoteur. Généralement, le gène est donc placé en aval du promoteur et en phase avec celui-ci. Par ailleurs, la région régulatrice est généralement placée en amont du promoteur, bien que, comme indiqué ci-avant, ce ne soit pas nécessaire à l'activité. La distance entre la région régulatrice (séquences NRSE) et le promoteur est variable selon la nature des séquences utilisées et le nombre de répétitions du motif NRSE. Avantageusement, les séquences régulatrices sont placée à une distance inférieure à 2 kb du promoteur, de préférence à une distance inférieure à 1kb.

Selon un mode particulier de l'invention, les séquences NRSE sont associées à des système de régulation afin de contrôler finement l'expression d'acides nucléiques dans les cellules. Parmi les systèmes de régulation, on peut citer tout particulièrement le système utilisant d'une part le transactivateur tTA controlé par la tétracycline et d'autre part un promoteur tTA sensible comme cela est décrit dans la demande FR 98/140080 et incorporée à la présente demande par référence. Brièvement, l'acide nucléique comporte une première région comprenant un acide nucléique codant pour le transactivateur du système de régulation par la tétracycline (tTA) sous le contrôle d'un promoteur modéré ainsi qu'une deuxième région comprenant un acide nucléique d'intérêt sous le contrôle d'un promoteur sensible au tTA. Ce promoteur sensible peut être tout promoteur, même fort, dont l'activité est augmentée en présence dudit transactivateur. D'un point de vue structural, ce promoteur comprend dans sa séquence ou à une distance fonctionelle de celle-ci, au moins un site de liaison (ou région opératrice Op) du facteur tTA. De manière préférée les deux régions précédentes sont séparées. Pour la réalisation de l'invention, les séquences NRSE peuvent être placées an amont du promoteur tTA sensible bien que cela ne soit pas nécessaire à l'activité, y compris entre les deux régions précédemment décrites.

Ce mode particulier de l'invention permet avantageusement d'avoir en même temps non seulement une régulation de l'expression d'un acide nucléique par la tétracycline mais aussi une spécificité tissulaire de l'expression et notamment dans les cellules nerveuses, apportée par les séquences NRSE. En outre, cette régulation fine du promoteur assure une efficacité et une sécurité remarquable dans l'expression de transgènes, nécéssaires en thérapie génique.

La présente invention est également relative à des vecteurs comprenant un acide nucléique tel que défini ci-avant. Ce vecteur est avantageusement capable de transduire les cellules nerveuses de mammifère, notamment humaines mais il n'est pas nécéssaire qu'il possède un tropisme particulier pour les dites cellules. Ainsi, l'invention permet avantageusement l'utilisation de tout type de vecteur. Il peut s'agir d'un vecteur de type plasmidique (plasmide, épisome, chromosome artificiel, etc.) ou viral. Parmi ces derniers, on peut citer de manière privilégiée les vecteurs dérivés des adénovirus, des AAV et des virus de l'herpès, dont le tropisme pour les cellules et tissus nerveux a été fortement documenté dans l'art antérieur. On peut également citer d'autres virus tels que les rétrovirus ou les rhabdovirus. A cet égard, les exemples fournis ci-après démontrent que les séquences NRSE introduites dans des vecteurs viraux ont une activité très importante. Ainsi, de manière inattendue, lorsque des séquences NRSE et notamment 6 et 12 séquences ont été introduites dans un vecteur viral (adénovirus), elles induisent une réduction de 91 à 98 % respectivement de l'expression d'un transgène dans des cellules non-nerveuses in vitro et de 90 à 96% in vivo. Ces résultats montrent avantageusement qu'il est possible d'exprimer spécifiquement dans les cellules nerveuses un acide nucléique d'interêt sans expression dans les cellules non-nerveuses, selon un système simple, grâce à ces séquences de régulation. En outre, ces séquences NRSE présentent l'avantage par leur taille réduite d'être particulièrement adaptées pour la régulation de l'expression de gènes incorporés dans un vecteur dans lequel l'espace d'insertion de séquences régulatrices est limité. Aussi, il est maintenant possible d'envisager d'associer ces séquences à d'autres systèmes de régulation dans un même vecteur.

Un objet particulier de l'invention réside donc dans un virus recombinant défectif comprenant un acide nucléique d'intérêt sous le contrôle de séquences d'expression, caractérisé en ce que lesdites séquences d'expression comprennent un promoteur et une ou plusieurs séquences NRSE.

D'une manière générale, les virus recombinants de l'invention sont défectifs, c'est-à-dire incapables de réplication autonome dans une cellule. La production de virus recombinants défectifs est connue de l'homme du métier, ainsi qu'illustré par exemple dans GRAHAM F. et PREVEC L, (In: Methods in Molecular Biology (1991) MURRAY E.J. (ed), the Humana Press Inc, Clifton, NJ, chapter 11, pp109-128). Notamment, chacun de ces virus peut être fabriqué dans des lignées cellulaires d'encapsidation possédant les dites fonctions déficientes. De telles lignées ont été décrites dans la littérature (293 et dérivés par exemple).

Selon un mode de réalisation particulier de l'invention, le virus recombinant défectif est un adénovirus. En particulier, pour ce virus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande WO94/26914). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprennent les ITR, une séquence permettant l'encapsidation et un acide nucléique selon l'invention. Encore plus préférentiellement, dans le génome des adénovirus de l'invention, la région E1 au moins est non fonctionnelle. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes. D'autres régions peuvent également être modifiées, et notamment la région E3 (WO95/02697), E2 (WO94/28938), E4 (WO94/28152, WO94/12649, WO95/02697) et L5 (WO95/02697). Selon un mode préféré de mise en oeuvre, l'adénovirus selon l'invention comprend une délétion dans les régions E1 et E4. Selon un autre mode de réalisation préféré, il comprend une délétion dans région E1 au niveau de laquelle sont insérés la région E4 et la séquence nucléique de l'invention (Cf FR94 13355). Il peut s'agir également d'un adénovirus recombinant, défectif pour les régions E1 et/ou E2 et/ou E4 par exemple. Dans les virus de l'invention, la délétion dans la région E1 s'étend préférentiellement des nucléotides 455 à 3329 sur la séquence de l'adénovirus Ad5.

Les adénovirus recombinants défectifs selon l'invention peuvent être préparés par toute technique connue de l'homme du métier (Levrero et al., gène 101 (1991) 195, EP 185 573; Graham, EMBO J. 3 (1984) 2917). En particulier, ils peuvent être préparés par recombinaison homologue entre un adénovirus et un plasmide portant entre autre un acide nucléique de l'invention ou un acide nucléique d'intérêt sous le contrôle de séquences d'expression comprenant un promoteur et une ou plusieurs séquences NRSE. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %) ou des lignées capables de complémenter les fonctions E1 et E4 telles que décrites notamment dans les demandes n° WO 94/26914 et WO95/02697 ou dans Yeh et al., J. Virol. 70 (1996) 559.
Ensuite, les adénovirus qui se sont multipliés sont récupérés et purifiés selon les techniques classiques de biologie moléculaire.

Selon un autre mode de réalisation particulier de l'invention, le virus recombinant défectif est un AAV. Les virus adéno-associés (AAV), sont des virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.
L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention sont défectifs pour tout ou partie des régions Rep et/ou Cap. Ils peuvent être préparés par co-transfection, dans un lignée cellulaire infectée par un virus auxiliaire humain (par exemple un adénovirus), d'un plasmide contenant une séquence nucléique ou une combinaison de séquences nucléiques de l'invention bordée de deux régions répétées inversées (ITR) d'AAV, et d'un plasmide portant les gènes d'encapsidation (gènes rep et cap) d'AAV. Une lignée cellulaire utilisable est par exemple la lignée 293. D'autres systemes de production sont decrits par exemple dans les demandes WO95/14771; WO95/13365; WO95/13392 ou WO95/06743. Les AAV recombinants produits sont ensuite purifiés par des techniques classiques.

Selon un autre mode de réalisation particulier de l'invention, le virus recombinant défectif est un rétrovirus, un rhabdovirus ou encore un HSV. Concernant les rétrovirus et les virus de l'herpès, la construction de vecteurs recombinants a été largement décrite dans la littérature : voir notamment Breakfield et al., New Biologist 3 (1991) 203; EP 453242, EP178220, Bernstein et al. gènet. Eng. 7 (1985) 235; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine Moloney leukemia virus"; encore désigné MoMLV), le MSV ("murine Moloney sarcoma virus"), le HaSV ("Harvey sarcoma virus"); le SNV ("spleen necrosis virus"); le RSV ("Rous sarcoma virus") ou encore le virus de Friend.

Pour construire des rétrovirus recombinants selon l'invention comportant un acide nucléique de l'invention ou un acide nucléique d'intérêt sous le contrôle de séquences d'expression comprenant un promoteur et une ou plusieurs séquences NRSE, un plasmide comportant notamment les LTR, la séquence d'encapsidation et ladite séquence nucléique est construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US4,861,719); la lignée PsiCRIP (WO90/02806) et la lignée GP+envAm-12 (WO89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

La présente demande montre la possibilité d'utiliser des séquences NRSE pour réguler l'expression d'un gène in vivo. En effet, les résultats présentés dans les exemples ci-après montrent que les séquences sont toujours activent in vivo et permettent une expression d'un transgène dans les cellules neuronales sans avoir d'expression dans les cellules non neuronales.

La demande montre également que les séquences NRSE sont capables, selon leur agencement (nombre de copies), de réguler efficacement l'activité d'un promoteur fort, ce qui permet des utilisations nombreuses et particulièrement avantageuses. En effet, les résultats obtenus démontrent que l'expression dans les cellules neuronales est non seulement specifique mais également comparable à celle obtenue avec des promoteurs forts. L'invention montre en outre de manière étonnante que l'activité des séquences NRSE semble être potentialisée lorsque celles-ci sont insérées dans un vecteur d'origine virale. Ce dernier type de construction combine donc des propriétés particulièrement attrayantes telles que l'efficacité du transfert et la sélectivité d'expression.

L'invention concerne également toute cellule comprenant un acide nucléique ou un vecteur ou un virus tel que défini ci-avant. Avantageusement, cette cellule est une cellule nerveuse de mammifère. Il peut s'agir en particulier d'une cellule provenant d'une lignée établie ou d'une cellule provenant d'une culture primaire. Ces cellules peuvent être utilisées pour la production de polypeptides par exemple, ou pour tester l'activité de gènes. Elles peuvent également être utilisées dans des approches de thérapie cellulaire, par implantation ou injection chez un sujet.

A cet égard, l'invention a aussi pour objet une composition comprenant un acide nucléique ou un vecteur ou un virus ou une cellule tels que définis ci-avant et un excipient. Avantageusement, il s'agit d'une composition pharmaceutique. Pour leur utilisation selon la présente invention, l'acide nucléique, le vecteur ou les cellules sont préférentiellement associés à un ou des véhicules pharmaceutiquement acceptables pour être formulé en vue d'administrations par voie topique et notamment stéréotaxique, orale, parentérale, intranasale, intraveineuse, intramusculaire, sous-cutanée, intraoculaire, transdermique, etc.... De préférence, l'acide nucléique, le vecteur ou les cellules sont utilisés sous une forme injectable. Il peut s'agir en particulier de solutions salines (phosphate monosodique, disodique, chlorure de sodium, potassium, calcium ou magnésium, etc, ou des mélanges de tels sels), stériles, isotoniques, ou de compositions sèches, notamment lyophilisées, qui, par addition selon le cas d'eau stérilisée ou de sérum physiologique, permettent la constitution de solutés injectables.

Selon un mode préféré, la composition selon l'invention est administrée par la voie intramusculaire, de préférence par injection. En effet, la voie intramusculaire choisie permet d'obtenir, de façon inattendue, un effet thérapeutique important, grâce au transport rétrograde des produits issus des gènes thérapeutiques et produits dans le muscle. En effet, ces produits, correspondant aux acides nucléiques ou issus des vecteurs selon l'invention, sont absorbés au niveau des jonctions neuromusculaires (plaques motrices) et sont acheminées jusqu'aux corps cellulaires des motoneurones par transport rétrograde le long des axones motoneuronaux. En outre, ce mode d'administration présente également l'avantage de prévenir les effets indésirables due à l'expression ectopique de gènes thérapeutiques dans le traitement des maladies neurologiques. Par cette méthode il devient donc aisé d'infecter spécifiquement les cellules neuronales sans promouvoir l'expression de transgènes dans les muscles injectés ou l'excrétion de leurs produits dans la circulation sanguine. En effet, elle palie incontestablement tous les inconvénients rencontrés jusqu'a présent en thérapie dûs à l'accès limité des facteurs neurotrophiques aux motoneurones, à la demi-vie très courte de ces protéines ainsi qu'aux effets délétères encourus lors d'une administration systémique de ces produits. Par ailleurs, étant donné la facilité d'accès du lieu à injecter, cette méthode est avantageusement utilisable quelque soit le type de pathologie neuronale ou motoneuronale.

Les quantités de ces différents éléments peuvent être ajustés par l'homme du métier en fonction des applications envisagées et en fonction de différents paramètres, comme notamment le site d'administration considéré, le nombre d'injections, le gène à exprimer, ou encore la durée du traitement recherchée.

L'invention concerne encore l'utilisation, pour la préparation d'une composition destinée au transfert et à l'expression d'un acide nucléique d'intérêt dans un tissu ou cellule nerveuse, d'une construction comprenant:
- un promoteur
- une ou plusieurs séquences NRSE, et
- ledit acide nucléique,
agencés de sorte que l'expression dudit acide nucléique soit contrôlée par ledit promoteur et que l'activité dudit promoteur soit contrôlée par la ou lesdites séquences.

L'utilisation des acides nucléiques de l'invention pour le transfert de gènes in vivo ou ex vivo, par exemple en thérapie génique, permet de limiter l'expression ectopique des transgènes d'intérêt et de cibler l'effet thérapeutique recherché aux populations neuronales. Elle empêche ainsi les effets délétères éventuels dus à la diffusion du transgène dans l'organisme. On peut envisager l'utilisation de ce système dans les différentes pathologies de la moelle épinière (dégénératives ou traumatiques) ainsi que dans toute autre pathologie neurologique centrale, périphérique ou neuropsychiatrique atteignant spécifiquement des populations neuronales. En neurologie fondamentale, ce système devrait également permettre de préciser l'origine (neuronale ou gliale) des effets observés in vitro et in vivo.

L'invention concerne également l'utilisation, pour la préparation d'une composition destinée au traitement des maladies motoneuronales, par voie intramusculaire, d'un acide nucléique d'intérêt dans un tissu ou cellule nerveuse, d'une construction comprenant:
- un promoteur
- une ou plusieurs séquences NRSE, et
- ledit acide nucléique,
agencés de sorte que l'expression dudit acide nucléique soit contrôlée par ledit promoteur et que l'activité dudit promoteur soit contrôlée par la ou lesdites séquences.

L'invention concerne également l'utilisation des vecteurs tel que définis précédemment et comprenant un acide nucléique d'intérêt sous le contrôle de séquences d'expression, caractérisé en ce que lesdites séquences d'expression comprennent un promoteur et une ou plusieurs séquences NRSE.

L'utilisation de vecteurs viraux repose sur les propriétés naturelles de transfection des virus. Ces vecteurs s'avèrent particulièrement performants sur le plan de la transfection. Il est ainsi possible d'utiliser les vecteurs de l'invention pour le transfert et l'expression dans les cellules neuronales de gène d'intérêt, in vivo, in vitro ou ex-vivo et notamment pour la thérapie génique. Ainsi, l'expression d'un acide nucléique d'interêt sous le contrôle notamment d'une ou plusieurs séquences NRSE est obtenue de manière spécifique dans les cellules nerveuses tandis que l'expression ectopique est limitée. Ces vecteurs peuvent donc être utilisés dans le traitement et/ou la prévention de différentes pathologies neurologiques centrales, périphériques ou neuropsychiatriques atteignant les cellules nerveuses et notamment les maladies neurodégénératives ainsi que différentes pathologies de la moelle épinière. A titre d'exemple, on peut citer particulièrement la maladie d'Alzheimer, la maladie de Parkinson, l'Ataxie spinale musculaire, la chorée d'Huntington.

Ces vecteurs peuvent notamment être utilisés dans le traitement et/ou la prévention des pathologies motoneuronales telles que par exemple la sclérose latérale amyotrophique, l'amyotrophie spinale de type I (maladie de Werdnig Hoffman), de type II ou III (maladie de Kugelberg-Welander), l'amyotrophie spinale bulbaire (telle que la maladie de Kennedy).

Ces vecteurs sont particulièrement adaptés au traitement et/ou à la prévention de ces pathologies par voie intramusculaire. Comme explicité précédemment cette voie thérapeutique permet d'atteindre les motoneurones grâce au transport rétrograde.

Plus précisément, l'invention concerne l'utilisation, pour la préparation d'une composition destinée au transfert et à l'expression d'un acide nucléique d'intérêt dans un tissu ou cellule nerveuse, d'un vecteur tel que défini précédemment et comprenant une construction dans laquelle :
- un promoteur,
- une ou plusieurs séquences NRSE, et
- ledit acide nucléique, sont agencés de sorte que l'expression dudit acide nucléique soit contrôlée par ledit promoteur et que l'activité dudit promoteur soit contrôlée par la ou lesdites séquences.

L'invention concerne également l'utilisation, pour la préparation d'une composition destinée au traitement des maladies motoneuronales, par voie intramusculaire, d'un acide nucléique d'intérêt dans un tissu ou cellule nerveuse, d'un vecteur tel que défini précédemment et comprenant une construction dans laquelle :
- un promoteur,
- une ou plusieurs séquences NRSE, et
- ledit acide nucléique, sont agencés de sorte que l'expression dudit acide nucléique soit contrôlée par ledit promoteur et que l'activité dudit promoteur soit contrôlée par la ou lesdites séquences.

L'invention concerne encore une méthode de régulation de l'expression de gènes in vivo comprenant l'insertion, en amont dudit gène, de séquences NRSE et l'administration in vivo de la construction résultante et/ou du vecteur comprenant ladite construction.

La présente demande sera décrite plus en détails à l'aide des exemples qui suivent, qui doivent être considérés comme illustratifs et non limitatifs. Ces exemples décrivent dans un premier temps le clonage d'un nombre croissant de séquences NRSE en amont du promoteur PGK dans des plasmides contenant le gène rapporteur de la luciférase, et la sélection des constructions permettant une diminution de l'expression de la luciférase après transfection de cellules non-neuronales. Dans un second temps, des constructions adénovirales correspondantes ont été générées et testées in vitro par infection de lignées cellulaires et de cultures primaires, puis in vivo après injection intramusculaire des adénovirus recombinants chez la souris. Les résultats présentés illustrent les avantages des acides nucléiques de l'invention.

### LÉGENDE DES FIGURES

Figure 1 : Représentation schématique des plasmides portant un promoteur chimérique de l'invention.

Figure 2 : Etude de fonctionalité des plasmides par transfection dans des cellules nerveuses (PC12) (Fig. 2B) et non-nerveuses (3T3) (Fig. 2A) puis mesure de l'activité luciférase.

Figure 3 :Etude de fonctionalité des virus recombinants défectifs in vitro par mesure de l'activité luciférase après infection :
- de cellules non-nerveuses (Fibroblastes de rat 3T3) (Fig 3A)
- de cellules nerveuses (Phéochromocytome de rat PC12) (Fig 3B)
- cultures de cellules primaires non-nerveuses, infection de cultures primaires de reins de rats nouveaux-nés (Fig 3C)
- de cultures de cellules primaires nerveuses, infection de cultures primaires de ganglions cervicaux supérieurs issus de rats nouveaux-nés (Fig 3D)
- de cultures de cellules primaires non-nerveuses, infection de cultures primaires d'astrocytes issus d'embryons de rats (Fig 3E)
- de cultures de cellules primaires nerveuses, infection de cultures primaires de cellules neuronales corticales issus d'embryons de rats (Fig 3F)

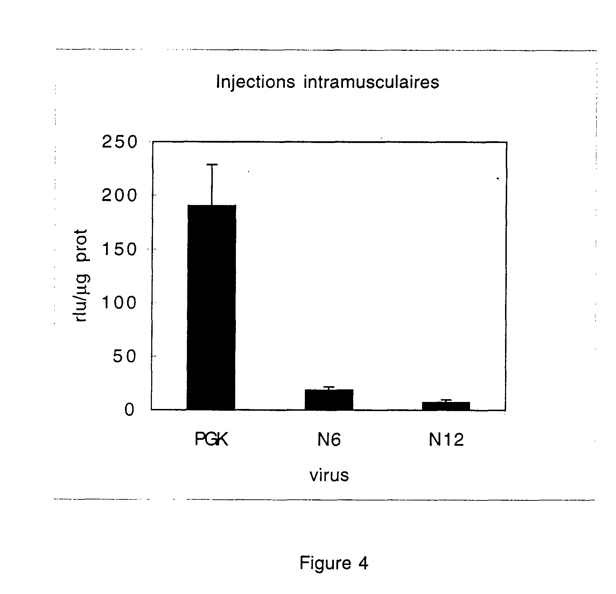

Figure 4: Etude de fonctionalité des virus recombinants défectifs in vivo par injection intramusculaire chez la souris et mesure de l'activité luciférase.

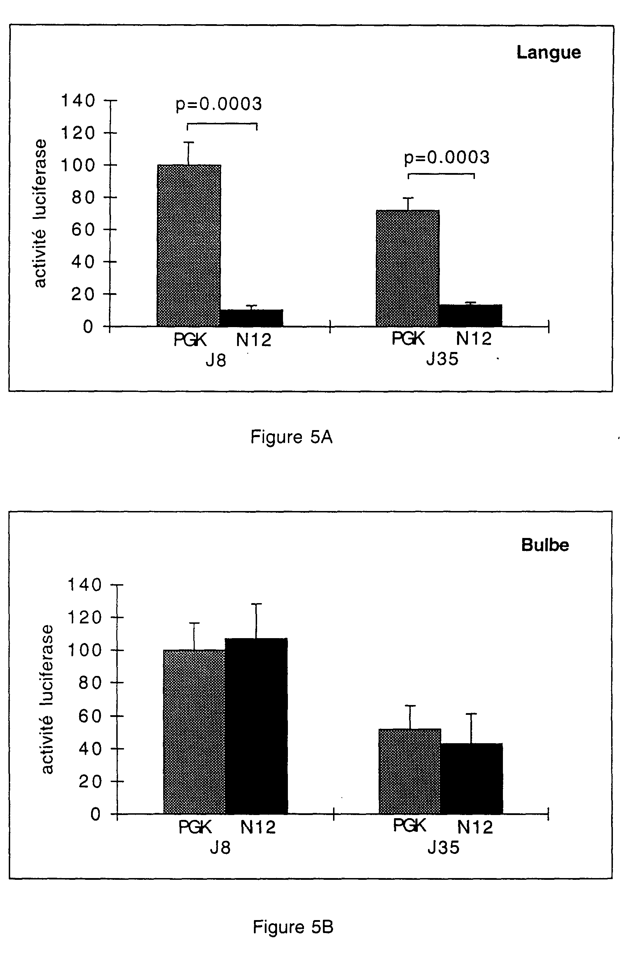

Figure 5 : Etude de fonctionalité des virus recombinants défectifs in vivo par injection dans les mucles linguaux chez la souris et mesure de l'activité luciférase
Fig.5 A: mesure de l'activité luciférase dans les cellules des muscles linguaux aux jours 8 et 35.
Fig. 5 B: mesure de l'activité luciférase dans les cellules du système nerveux (bulbe) aux jours 8 et 35.

### MATERIELS ET METHODE

### Lignées cellulaires et cultures primaires :

Les cellules issues de phéochromocytome de rat (PC12) sont cultivées en milieu RPMI 1640 (Gibco) contenant 15% de serum de veau foetal (SVF) (Boehringer). Les cellules 3T3 issues de fibroblates de rat sont maintenues en milieu Dulbecco's modifié Eagle's (DMEM) contenant 10% de serum de veau foetal. Les cultures primaires de ganglion cervical supérieur (GCS) sont obtenues à partir de dissections de ganglions cervicaux supérieurs de rats Winstar (Iffa-Credo) nouveaux-nés, agés de 1 ou 2 jours et dissociés par 3mg/ml de dispase (Boehringer), déposés ensuite sur des plaques préalablement recouvertes de collagène issu de queue de rat. Les cellules sont cultivées dans 0,5 ml de milieu Leibovitz's L-15 (Gibco) tamponé au bicarbonate et contenant plusieurs facteurs dont 70 ng/ml de NGF (nerve growth Factor), 5% de sérum de rat adulte et 10µM de cytosine arabinofuranoside.

Les cultures primaires de rein sont préparées par dissociation à la trypsine de tissu rénal de rats Winstar (Iffa-Credo) nouveaux-nés, agés de 1 ou 2 jours. Les cellules sont ensuite déposées en plaques dans 10% de SVF afin de permettre des divisions cellulaires jusqu'à l'infection.

Les cultures corticales sont issues d'embryons de rat E17 Sprague-Dawley (Iffa-Credo). Les cellules sont obtenues par dissection et dissociation mécanique et cultivées en milieur DMEM contenant 100 µg/ml de transferrine, 25µg/ml d'insuline, 10µg/ml de putrescine, 5 ng/ml de selenite de sodium, 6,3 ng/ml de progestérone et 2mM de glutamine (Sigma).

Les cultures primaires d'astrocytes sont dérivées de tissu cortical embryonnaire de rat (E17 Sprague-Dawley) et cultivées en milieu DMEM contenant 10 % de SVF dans une atmosphère de 10% de CO₂ et 90 % d'air.

### Expériences in vitro :

Pour les expérience de transfection transitoire, un million de cellules sont électroporées au moyen d'unsystème Bio-Rad à 960µF et 190V pour les cellules PC12 ou 250V pour les cellules 3T3 avec 6µg de chaque plasmides testés, 7 µg de plasmide Blue Script (Stratagène) et 2 µg de vecteur pCAT 3- contrôle (Promega) qui est un plasmide codant pour la Chloramphénicol Acétyl Transférase (CAT) permettant le contrôle de l'efficacité de la transfection.

Les cultures cellulaires sont infectées en remplaçant le milieu de culture par du milieu sans serum contenant la suspension virale à une MOI de 200 pfu environ et en incubant pendant 45 minutes. Pour chaque construction (plasmidique ou adénovirale) 3 valeurs d'activité luciférase sont déterminées sur 3 puits différents. Les expériences d'infection sont répétées deux fois avec des stocks viraux différents pour chaque construction virale.

Les cellules sont récoltées 48 heures après l'électroporation ou l'infection dans 200 µl d'un tampon de lyse contenant 25 mM de Tris phosphate pH 7,8; 0,08 mM de Luciférine; 0,1 mM d'ATP; 8 mM de MgCl₂; 1 mM de dithiothreitol; 1 mM d'EDTA; 15% de glycérol et 1 % de Triton. L'activité luciférase est mesurée à l'aide d'un luminomètre de type LUMAT LB9501 (Berthold). Pour les cellules tranfectées, 1% de serum albumine bovine (SAB) est ajouté au tampon de lyse et l'activité est normalisée sur la base de l'activité CAT déterminée par la méthode de comptage d'un liquide de scintillation. Pour les adénovirus recombinants, l'activité luciférase est normalisée en fonction de la concentration en protéine des extraits cellulaires, déterminée par le système Bio-Rad (Laboratoires Bio-Rad).

### Expériences in vivo :

Des souris mâle C57B16 (Charles River), agés de six mois sont anesthésiés avec un mélange de Rompun (Bayer)/Ketamine (UVA) et les constructions Ad-PGK-luc et Ad-NRSE-PGK-luc sont injectées lentement (2,5 µl/ml) dans la langue (10⁹ pfu/ 4x2,5µl) et/ou dans le muscle gastrocnémien à une dose de 2.10⁹ pfu/muscle (30µl).

Les souris sont sacrifiées avec du pentobarbital (Sanofi) 7 à 35 jours après l'infection. Les bulbes, les langues et les muscles sont prélévés pour mesurer l'activité luciférase.Les bulbes sont dissociés mécaniquement dans 200 µl de tampon de lyse. Les muscles et les langues sont dissociées à l'aide d'un système de type DIAX 900 polytron (Heidolph) dans 1 et 2 ml de tampon de lyse respectivement.

### EXEMPLES

### 1. Construction des promoteurs chimériques et des vecteurs plasmidiques

Le but de cet exemple est de décrire l'obtention des promoteurs chimériques ainsi que les contructions de vecteurs plasmidiques comportant ces promoteurs particuliers.

Le plasmide pPGK-Luc comprend le gène Luc sous contrôle du promoteur eucaryote ubiquitaire PGK. Pour obtenir le plasmide pPGK-Luc, les 500 pb du promoteur PGK murin ont été insérés dans un plasmide commercial pUT 103 (CAYLA FRANCE) comportant le gène rapporteur luciférase fusionné à la zéocine et une séquence de polyadénylation de SV-40 en configuration "early" (PolyA).

Ensuite, une, deux, trois, six et douze copies de la séquence NRSE du gène SCG10 (TTCAGCACCACGGAGAGTGCC, SEQ ID n° 1) [2] ont été insérées dans une configuration antiparallèle en amont du promoteur PGK. Pour cela, un fragment de 26 pb contenant la séquence NRSE décrite ci-dessus encadrée par 2 sites MluI a été construit par hybridation de 2 oligonucléotides synthétiques correspondants. Ce fragment a été inséré en une ou plusieurs copies au niveau du site MluI de pPGK-Luc et a permis l'obtention des plasmides comportant 1, 2 et 3 copies NRSE. Le plasmide comportant 6 copies NRSE a été obtenu à partir du plasmide en comportant 3 (pNRSE3-PGK-Luc) selon la stratégie suivante : un fragment BamHI / XhoI contenant 3 copies NRSE est extrait de pNRSE3-PGK-Luc et introduit au niveau du site XhoI de pNRSE3-PGK-Luc, ce qui génère p6NRSE-PGK-Luc. De même, le plasmide comportant 12 copies NRSE a été obtenu à partir du plasmide en comportant 6 (p6NRSE-PGK-Luc) en introduisant le fragment BamHI / XhoI en double copie dans p6NRSE-PGK-Luc.

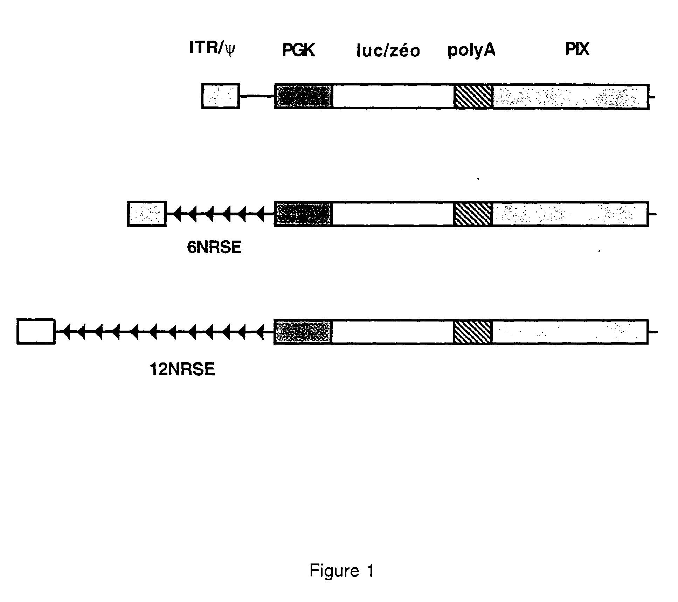

Enfin, la cassette d'expression PGK (avec ou sans NRSE)-Luc-PolyA des différents plasmides décrits ci-dessus a été introduite dans un plasmide navette utilisable pour la construction de virus recombinants défectifs, entre la séquence ITR gauche de l'adénovirus (Inverted Terminal Repeat, origine de réplication) et la séquence codant pour le polypeptide IX (protéine de la capside virale) (figure 1).

Il est entendu que, selon le même protocole, tout autre plasmide peut être construit incorporant de 1 à 50 copies d'un motif NRSE, associé à un promoteur ubiquitaire eucaryote autre que PGK, sous le contrôle duquel peut être inséré tout transgène d'intérêt.

### 2. Analyse fonctionnelle par transfections de lignées cellulaires

Le but de cet exemple est de démontrer que les contructions plasmidiques comportant le promoteur chimérique comprenant les séquences NRSE sont fonctionelles et permettent l'expression en cellule d'un gène d'intéret.

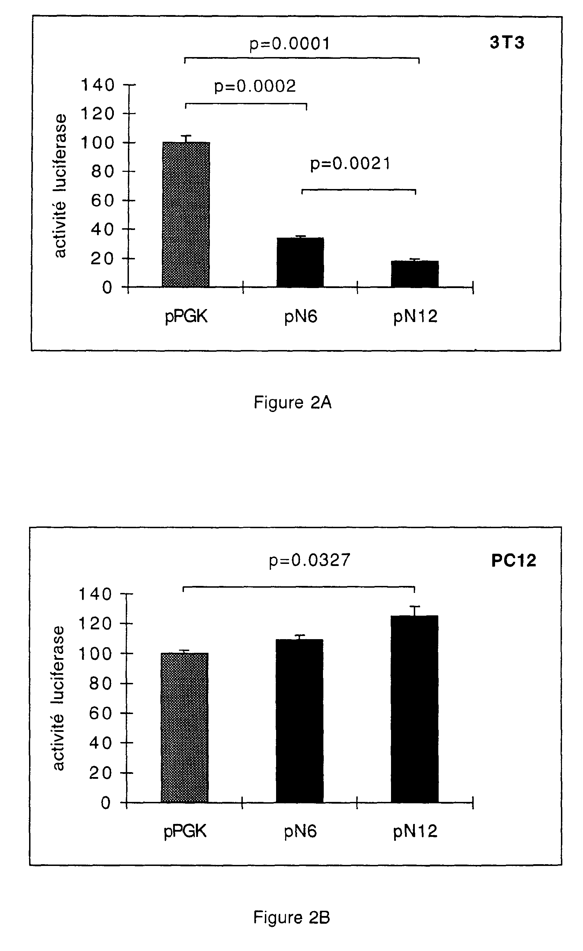

Les constructions plasmidiques décrites dans l'exemple 1 ont été testées par transfection transitoire (électroporation) de lignées neuronales PC12 (phéochromocytome de rat) et non neuronales 3T3 (fibroblastes de rat). Les valeurs d'activité luciférase obtenues sont normalisées par rapport à l'activité CAT (Chloramphénicol Acétyl Transférase) mesurée dans une solution contenant 125 mM de Tris phosphate pH 7,8, 125 µM de chloramphénicol et 0,12 µM d'acétylcoenzyme A radiomarqué. Les dosages sont réalisés en présence d'un fluide de scintillation Econofluor à l'aide d'un compteur KONTRON après une heure d'incubation à 37°C. Pour chaque construction plasmidique, 3 valeurs d'activité luciférase ont été déterminées et les moyennes et les erreurs standards obtenues sont présentées dans la figure 2.

Les résultats obtenus montrent que les constructions comportant 6 et 12 copies NRSE sont capables de diminuer l'expression de la luciférase de 66 et 82 % respectivement, dans les cellules non-nerveuses (3T3), soit une perte de près d'un logarithme d'expression par rapport à la construction pPGK-Luc. Les résultats montrent également que la présence des motifs NRSE dans les cellules nerveuses PC12 ne diminue pas l'expression de la luciférase, voire même est succeptible de l'augmenter. Une augmentation de 25% statistiquement significative est en effet observée entre p12NRSE-PGK-Luc et pPGK-Luc.

Ainsi, il est démontré par les résultats obtenus que les contructions plasmidiques comportant le promoteur chimérique comprenant les séquences NRSE sont fonctionelles et permettent l'expression en cellule d'un gène d'intéret dans les cellules neuronales uniquement tout en inhibant de manière très significative l'expression dudit gène dans les non neuronales.

### 3. Construction de virus recombinants défectifs

Le but de cet exemple est de décrire l'obtention des promoteurs chimériques ainsi que les contructions de vecteurs viraux comportant ces promoteurs particuliers.

Dans le but de compléter cette étude in vitro et in vivo, les trois constructions adénovirales correspondantes ont été générées : Ad-PGK-Luc, Ad-6NRSE-PGK-Luc et Ad-12NRSE-PGK-Luc. Ces virus recombinants ont été construits selon les techniques connues de biologie, par co-transfection, dans une lignée d'encapsidation (cellules 293) des plasmides navettes décrits dans l'exemple 1 et d'un génome adénoviral défectif de type 5. Les plasmides navette ont tout d'abord été linéarisés par coupure enzymatique au site *Fsp*I et co-transfectés avec le long fragment *Cla*I de l'adénovirus dans la lignée cellulaire 293 par la méthode de précipitation de l'ADN au phosphate de calcium.

Les génomes recombinants encapsidés sont ensuite purifiés selon les techniques classiques et notamment par la technique de purification en plaques. Le fragment intégré est ensuite vérifié par analyse des fragments de restriction et par PCR. Les stocks de virus ont été préparés par propagation de l'adénovirus recombinant en cellule 293 puis par ultracentrifugation notamment en gradient de chlorure de Césium et purification sur colonne de purification de type Sephadex G25M (Pharmacia). Les titres viraux ont été déterminé par lecture de la densité optique et vérifié en outre par la méthode en plaque. Tous les stocks de virus avaient des titre autour de 2.10⁸ pfu/µl.

### 4. Analyse fonctionnelle in vitro

Le but de cet exemple est de démontrer que les contructions virales décrites dans l'exemple 3 et comportant le promoteur chimérique comprenant les séquences NRSE sont fonctionelles et permettent le contrôle du promoteur dans différents types cellulaires.

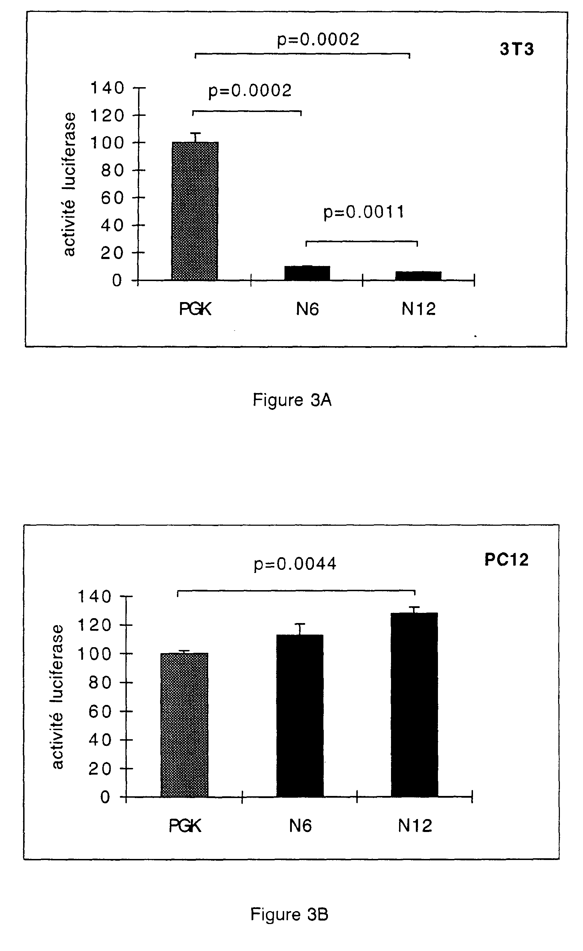

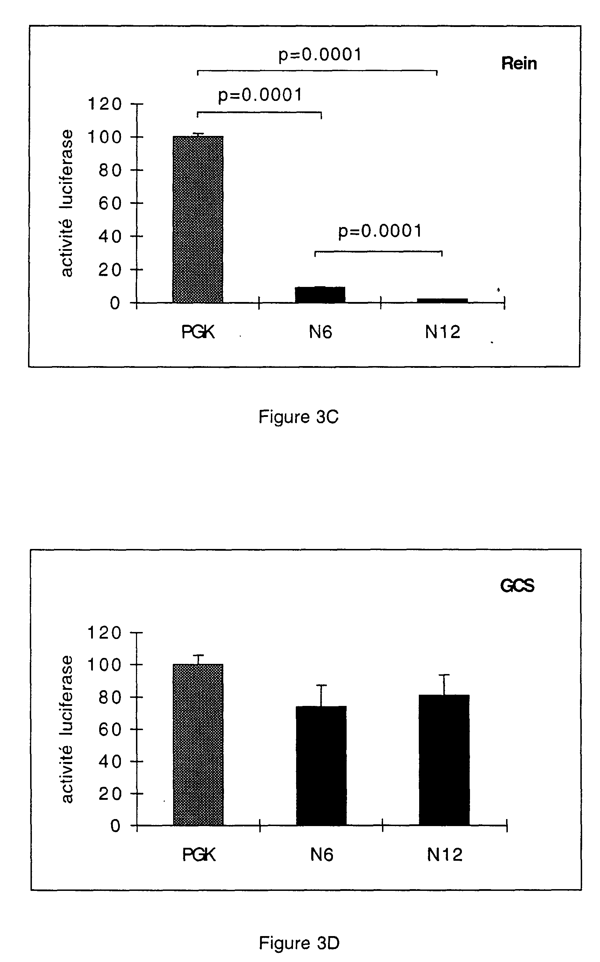

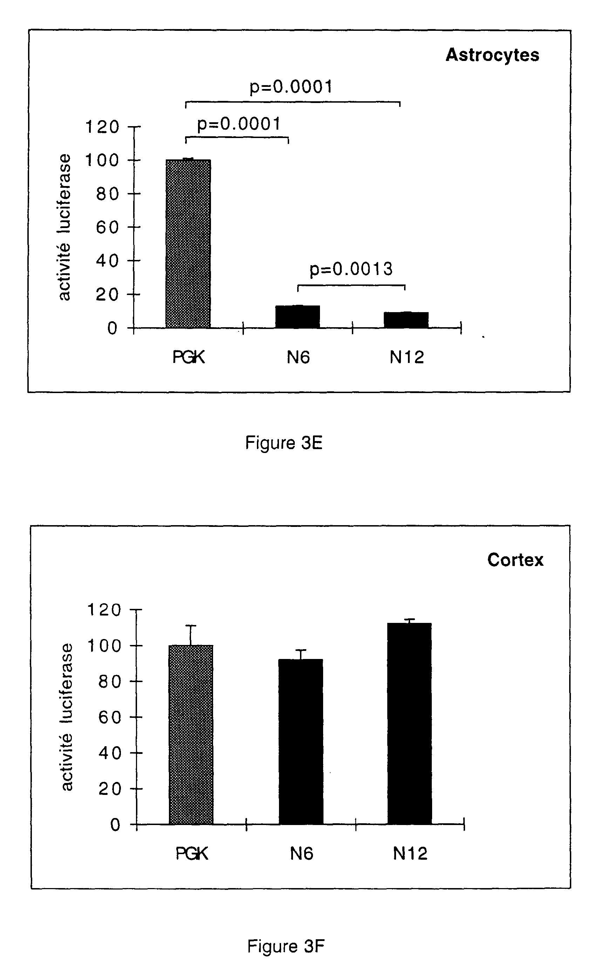

Les trois adénovirus construits dans l'exemple 3 ci-dessus ont été testés in vitro par infection de lignées cellulaires et de cultures primaires à une dose de 20 et 200 pfu/cellule respectivement. Les mesures sont effectuées comme décrit précédemment sur 50 µl de surnageant et normalisées par rapport à la quantité de protéines totales, obtenue à l'aide d'un système Bio Rad protéin assay kit (Bio-Rad). Pour chaque adénovirus, 3 valeurs d'activité luciférase ont été déterminées sur des puits différents. Les valeurs moyennes et les erreurs standards obtenues sont illustrées figure 3 pour les lignées et figure 4 pour les cultures primaires.

L'expression du gène rapporteur luciférase est diminuée de 97 % et 99 % respectivement avec les adénovirus recombinants Ad-6NRSE-PGK-Luc et Ad-12NRSE-PGK-Luc dans la lignée non-neuronale 3T3.

Dans la lignée neuronale PC12, l'activité luciférase des deux adénovirus comportant des séquences NRSE est supérieure à celle observée avec l'adénovirus Ad-PGK-Luc.

De même, après infection de cultures primaires de ganglion cervical supérieur (GCS) de rats nouveaux-nés, l'activité luciférase est similaire pour les 3 constructions adénovirales (une analyse de variance globale à un facteur ANOVA ne montre pas de différence significative entre les trois groupes). En revanche, l'expression de la luciférase est diminuée de 91 à 98 % après infection des cultures primaires de reins de rats nouveaux-nés avec les adénovirus recombinants Ad-6NRSE-PGK-Luc et Ad-12NRSE-PGK-Luc, soit une perte de 2 logarithmes d'expression de PGK en présence des 12 copies NRSE.

Confirmant les résultats précédents, l'infection par les 3 constructions d'astrocytes et de cellules du cortex neuronal issues d'embryons de rats âgés de 17 jours, montre comme attendu que l'activité luciférase est très significativement diminuée (d'un facteur 10) dans les cellules astrocytaires pour les constructions comportant 6 ou 12 séquences NRSE (fig 3 E). Comme également prévu, l'activité luciférase'est comparable dans les cellules du cortex, pour les trois types de constructions virales précédemment citées (fig 3 F).

Ces résultats sont particulièrement surprenants et remarquables dans la mesure où ils montrent (i) que les promoteurs chimériques de l'invention sont fonctionnels sur des cultures primaires, (ii) que les promoteurs sont fonctionnels dans un contexte viral et (iii) que l'activité de répression est plus grande dans le contexte adénoviral que dans le vecteur plasmidique correspondant (Cf figures 2 et 3).

Ainsi, cet exemple a permis de démontrer que les contructions virales comportant le promoteur chimérique comprenant les séquences NRSE sont fonctionelles et permettent l'expression en cellule d'un gène d'intéret dans les cellules neuronales uniquement tout en inhibant de manière très significative l'expression dudit gène dans les cellules non neuronales.

### 5. Analyse fonctionnelle in vivo

Le but de cet exemple est de démontrer que les contructions virales décrites dans l'exemple 3 et comportant le promoteur chimérique comprenant les séquences NRSE sont fonctionelles et permettent le contrôle in vivo du promoteur .

Compte tenu des résultats très encourageants décrits dans l'exemple 4, l'activité des constructions de l'invention a été testée in vivo, par mesure de l'expression de la luciférase après injection intramusculaire (muscle gastrocnémien et muscle de la langue) de ces 3 adénovirus recombinants chez la souris.

### 5.1 Injection dans le muscle gastrocnémien

Chaque construction adénovirale a été injectée en 3 points dans le muscle gastrocnémien droit de 7 souris à une dose de 2.10⁹ pfu/muscle, sous un volume de 30 µl. Les muscles injectés ont été prélevés 7 jours après l'injection et dissociés dans 1 ml de tampon en vue d'une détermination de l'activité luciférase réalisée sur 150 µl de surnageant. Les moyennes et les erreurs standards des 7 valeurs d'activités luciférase obtenues, rapportées à la quantité de protéine, sont présentées sur la figure 5.

Ces résultats montrent que l'expression de la luciférase est diminuée de 90 et 96 % avec les adénovirus recombinants Ad-6NRSE-PGK-Luc et Ad-12NRSE-PGK-Luc une semaine après les injections.

L'expression de la luciférase après injection intra-musculaire de Ad-12NRSE-PGK-Luc et de Ad-PGK-Luc a été comparée a plus long terme chez la souris. Une diminution de l'activité luciférase est toujours observée avec Ad-12NRSE-PGK-Luc un mois (95%) et trois mois (91%) après les injections.

### 5.2 Injection dans les muscles de la langue

Le transport rétrograde a été utilisé afin de comparer l'expression des deux constructions Ad-PGK-Luc et Ad-12NRSE-PGK-Luc dans le bulbe et dans les muscles de la langue après injection intramusculaire dans la langue de souris.

De manière comparable à ceux déjà obtenus, les activités luciférases des deux constructions sont similaires dans le système nerveux, une semaine après l'administration (fig.5 B). A l'inverse, l'activité luciférase diminue de 90 % dans les muscles injectés avec la construction Ad-12NRSE-PGK-Luc par rapport aux muscles injectés avec la construction Ad-PGK-Luc (fig.5 A). Cette répression est maintenue dans le temps et pendant au moins 35 jours après l'infection. De manière intéressante, bien que le niveau d'expression à 35 jours soit inférieur à celui obtenu à 8 jours après l'infection, la différence d'expression entre les deux constructions virales précédentes demeure très significative.

Une fois encore, ces résultats confirment la fonctionalité des constructions de l'invention in vivo, et font apparaitre un facteur de répression encore supérieur à ceux observés in vitro après transfection des plasmides dans les cellules non neuronales.

Ces résultats démontrent en outre, la haute efficacité des constructions de l'invention in vivo pour cibler l'expression d'un gène d'intérêt dans les cellules neuronales. L'utilisation de cette approche en thérapie génique permet de limiter l'expression ectopiques des transgènes d'intérêt et d'empêcher les effets délétères éventuels dus à la diffusion du transgène dans l'organisme.

### BIBLIOGRAPHIE

[1] Mori N., Stein R., Sigmund O. And Anderson D.J.
   Neuron, 4 : 583-594 (1990)
[2] Mori N., Schoenherr C., Vandenbergh D.J. And Anderson D.J.
   Neuron, 9 : 45-54 (1992)
[3] Li L., Suzuki T., Mori N. And Greencard P.
   PNAS USA, 90 : 1460-1464 (1993)
[4] Schoenherr C.J., Paquette A.J. and Anderson D.J.
   PNAS USA, 93 : 9981-9886 (1996)
[5] Schoenherr C.J. and Anderson D.J.
   Science, 267 : 1360-1363 (1995)
[6] McBurney, Sutherland, Adra, Leclair, Rudnicki, Jardine
   Nucleic Acids Research, Vol.19, N° 20 : 5755-5761 (1991)
[7] Moullier P., Marechal V., Danos O. And Heard J.M.
   Transplantation, 56 : 427-432 (1993)
[8] McDonald R.J., Lukason M.J., Raabe O.G., Canfield D.R., Burr
   E.A., Kaplan J.M., Wadsworth S.C. and St George J.A.
   Hum. Gene Ther., 8 : 411-422 (1997)
[9] Maue R.A., Kraner S.D., Goodman R.H. and Mandel G.
   Neuron, 4 : 223-231 (1990).
[10] Bessis A., Champtiaux N., Chatelin L. and Changeux J.P.
   PNAS USA, 94 : 5906-5911 (1997).

## Revendications

1. Acide nucléique recombinant comprenant:
- un promoteur
- une ou plusieurs séquences NRSE, et
- un gène thérapeutique,
agencés de sorte que l'expression dudit gène thérapeutique soit contrôlée par ledit promoteur et que l'activité dudit promoteur soit contrôlée par la ou lesdites séquences.

2. Acide nucléique recombinant selon la revendication 1 caractérisé en ce que le promoteur est un promoteur eucaryote ou viral actif dans les cellules ou le tissus nerveux.

3. Acide nucléique recombinant selon la revendication 2 caractérisé en ce que le promoteur est un promoteur eucaryote ubiquitaire, neurospécifique ou spécifique à un type neuronal.

4. Acide nucléique recombinant selon la revendication 3 caractérisé en ce que le promoteur est un promoteur domestique.

5. Acide nucléique recombinant selon la revendication 4 caractérisé en ce que le promoteur est choisi parmi le promoteur des gènes PGK, Ef1α, βactine, vimentine, aldolase A ou α1-antitrypsine.

6. Acide nucléique recombinant selon la revendication 1 caractérisé en ce que le promoteur est un promoteur dit fort.

7. Acide nucléique recombinant selon la revendication 1 caractérisé en ce qu'il comprend de 1 à 20 séquences NRSE, de préférence de 3 à 15.

8. Acide nucléique recombinant selon la revendication 7 caractérisé en ce qu'il comprend de 3, 6 ou 12 séquences NRSE.

9. Acide nucléique recombinant selon la revendication 8 caractérisé en ce que la séquence NRSE comprend tout ou partie de la séquence SEQ ID n° 3 dans laquelle N vaut A, G, C ou T.

10. Acide nucléique recombinant selon la revendication 9 caractérisé en ce que la séquence NRSE est choisie parmi tout ou partie des séquences SEQ ID n° 1, 2, 4-11 ou de variants de celles-ci.

11. Acide nucléique recombinant selon l'une des revendications précédentes caractérisé en ce qu'il comporte soit le même motif soit des motifs variants définis selon la revendication 9 ou 10, répétés plusieurs fois.

12. Acide nucléique recombinant selon l'une des revendications précédentes caractérisé en ce que la ou les séquences NRSE sont placées en amont du promoteur.

13. Acide nucléique recombinant selon la revendication 1 caractérisé en ce que le gène thérapeutique est un acide nucléique comprenant une phase ouverte de lecture codant pour un ARN ou un polypeptide thérapeutique ou vaccinal.

14. Acide nucléique recombinant selon la revendication 13 caractérisé en ce que le gène thérapeutique est un acide nucléique comprenant une phase ouverte de lecture codant pour un facteur trophique.

15. Acide nucléique recombinant selon la revendication 13 caractérisé en ce que le gène thérapeutique est un acide nucléique comprenant une phase ouverte de lecture codant pour un agent antioxydant.

16. Acide nucléique recombinant selon la revendication 1 caractérisé en ce qu'il comprend des éléments de régulation en plus des séquences NRSE.

17. Eléments de régulation selon la revendication 16 caractérisé en ce qu'il s'agit d'un système tétracycline opérateur/répresseur.

18. Vecteur comprenant un acide nucléique selon l'une des revendications 1 à 16.

19. Vecteur selon la revendication 18 caractérisé en ce qu'il s'agit d'un vecteur viral.

20. Virus recombinant défectif comprenant un acide nucléique d'intérêt sous le contrôle de séquences d'expression, caractérisé en ce que lesdites séquences d'expression comprennent un promoteur et une ou plusieurs séquences NRSE.

21. Virus selon la revendication 20 caractérisé en ce qu'il s'agit d'un adénovirus.

22. Virus selon la revendication 20 caractérisé en ce qu'il s'agit d'un AAV.

23. Virus selon la revendication 20 caractérisé en ce qu'il s'agit d'un rétrovirus.

24. Virus selon la revendication 20 caractérisé en ce qu'il s'agit d'un rhabdovirus.

25. Cellule comprenant un acide nucléique selon la revendication 1 ou un vecteur selon la revendication 18 ou un virus selon la revendication 20.

26. Cellule selon la revendication 25 caractérisée en ce qu'il s'agit d'une cellule nerveuse de mammifère.

27. Utilisation, pour la préparation d'une composition destinée au transfert et à l'expression d'un acide nucléique d'intérêt dans un tissu ou cellule nerveuse, d'une construction comprenant:
- un promoteur
- une ou plusieurs séquences NRSE, et
- ledit acide nucléique,
agencés de sorte que l'expression dudit acide nucléique soit contrôlée par ledit promoteur et que l'activité dudit promoteur soit contrôlée par la ou lesdites séquences.

28. Utilisation, pour la préparation d'une composition destinée au traitement des maladies motoneuronales par voie intramusculaire, d'un acide nucléique d'intérêt dans un tissu ou cellule nerveuse, d'une construction comprenant:
- un promoteur
- une ou plusieurs séquences NRSE, et
- ledit acide nucléique,
agencés de sorte que l'expression dudit acide nucléique soit contrôlée par ledit promoteur et que l'activité dudit promoteur soit contrôlée par la ou lesdites séquences.

29. Utilisation d'un virus recombinant défectif comprenant un acide nucléique d'intérêt sous le contrôle de séquences d'expression, caractérisé en ce que lesdites séquences d'expression comprennent un promoteur et une ou plusieurs séquences NRSE.

30. Utilisation selon la revendication 29 pour le transfert et l'expression de gène d'intérêt dans les cellules neuronales in vivo, in vitro ou ex-vivo.

31. Utilisation, pour la préparation d'une composition destinée au transfert et à l'expression d'un acide nucléique d'intérêt dans un tissu ou cellule nerveuse, d'un vecteur comprenant une construction dans laquelle :
- un promoteur,
- une ou plusieurs séquences NRSE, et
- ledit acide nucléique, sont agencés de sorte que l'expression dudit acide nucléique soit contrôlée par ledit promoteur et que l'activité dudit promoteur soit contrôlée par la ou lesdites séquences.

32. Utilisation, pour la préparation d'une composition destinée au traitement des maladies motoneuronales, par voie intramusculaire, d'un vecteur comprenant une construction dans laquelle :
- un promoteur,
- une ou plusieurs séquences NRSE, et
- ledit acide nucléique, sont agencés de sorte que l'expression dudit acide nucléique soit contrôlée par ledit promoteur et que l'activité dudit promoteur soit contrôlée par la ou lesdites séquences.

33. Utilisation selon l'une des revendications 31 ou 32 caractérisée en ce que le vecteur est un virus.

34. Composition comprenant un acide nucléique selon la revendication 1 ou un vecteur selon la revendication 18 ou un virus selon la revendication 20.

35. Composition selon la revendication 34 caractérisée en ce qu'elle est formulée en vue d'être administrée par la voie intramusculaire, de préférence par injection.

36. Promoteur chimérique comprenant un promoteur ubiquitaire et une ou plusieurs séquences NRSE.

37. Promoteur chimérique xNRSE-PGK dans lequel x est un nombre entier de 1 à 50.


LISTE DE SEQUENCES

<110> RHONE-POULENC RORER S.A.

<120> Utilisation d'éléments de régulation négative pour
      l'expression neurospécifique de transgènes.

<130> ST98004

<140> PCT/FR99/00061
<141> 1999-01-14

<150> FR9801715
<151> 1998-02-12

<160> 11

<170> PatentIn Ver. 2.1

<210> 1
<211> 21
<212> ADN
<213> Séquence artificielle

<220>
<223> Description de la séquence artificielle:séquence
      du motif NRSE

<400> 1
ttcagcacca cggagagtgc c                                            21

<210> 2
<211> 21
<212> ADN
<213> Séquence artificielle

<220>
<223> Description de la séquence artificielle:séquence
      consensus du motif NRSE

<400> 2
ttcagcacca cggacagcgc c                                            21

<210> 3
<211> 21
<212> ADN
<213> Séquence artificielle

<220>
<223> Description de la séquence artificielle:séquence
      générale du motif NRSE

<400> 3
   nncagcaccn ngganagnnn c                                          21

<210> 4
<211> 21
<212> ADN
<213> Séquence artificielle

<220>
<223> Description de la séquence artificielle:variant du
      motif NRSE

<400> 4
ttcagcacca cggagagtgc c                                            21

<210> 5

<211> 21
<212> ADN
<213> Séquence artificielle

<220>
<223> Description de la séquence artificielle:variant du
      motif NRSE

<400> 5
ttcagcaccg cggacagtgc c                                                21

<210> 6
<211> 21
<212> ADN
<213> Séquence artificielle

<220>
<223> Description de la séquence artificielle:variant du
      motif NRSE

<400> 6
ttcagcacct cggacagcat c                                                21

<210> 7
<211> 21
<212> ADN
<213> Séquence artificielle

<220>
<223> Description de la séquence artificielle:variant du
      motif NRSE

<400> 7
ttcagcaccg cggagagcgt c                                                21

<210> 8
<211> 21
<212> ADN
<213> Séquence artificielle

<220>
<223> Description de la séquence artificielle:variant du
      motif NRSE

<400> 8
tccagcaccg tggacagagc c                                                21

<210> 9
<211> 21
<212> ADN
<213> Séquence artificielle

<220>
<223> Description de la séquence artificielle:variant du
      motif NRSE

<400> 9
ttcagcaccg aggacggcgg a                                                21

<210> 10
<211> 21
<212> ADN
<213> Séquence artificielle

<220>
<223> Description de la séquence artificielle:variant du
      motif NRSE

<400> 10
atcagcacca cggacagcgg c                                                21

<210> 11
<211> 21
<212> ADN
<213> Séquence artificielle

<220>
<223> Description de la séquence artificielle:variant du
      motif NRSE

<400> 11
ttcagcacct aggacagagg c                                                21